(19) Europäisches Patentamt
European Patent Office
Office européen des brevets

(11) **EP 1 739 623 A1**

(12) **EUROPEAN PATENT APPLICATION**

(43) Date of publication:
**03.01.2007 Bulletin 2007/01**

(51) Int Cl.:
***G06T 7/20*** (2006.01)    ***A61B 8/00*** (2006.01)

(21) Application number: **06013450.9**

(22) Date of filing: **29.06.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **01.07.2005 KR 20050058972**

(71) Applicant: **MEDISON CO., LTD.**
**Kangwon-do 250-870 (KR)**

(72) Inventors:
• **Kim, Baek Sop**
  **Chuncheon-si**
  **Gangwon-do 200-170 (KR)**

• **Kim, Jong Dae**
  **Gangnam-gu**
  **Seoul 135-828 (KR)**
• **Shin, Dong Kuk**
  **Gangnam-gu**
  **Seoul 135-280 (KR)**
• **Shin, Seong Chul**
  **Gangnam-gu**
  **Seoul 135-280 (KR)**

(74) Representative: **Schmid, Wolfgang**
**Lorenz & Kollegen**
**Patent- und Rechtsanwaltskanzlei**
**Alte Ulmer Strasse 2**
**D-89522 Heidenheim (DE)**

(54) **Hierarchical motion estimation method and ultrasound imaging system using the same**

(57)    The present invention relates to a hierarchical motion estimation method and an ultrasound imaging system using the same. The hierarchical motion estimation method includes decomposing a first input image and a second input image into at least two hierarchical, multi-resolution images (S110); selecting one of the multi-resolution images decomposed from the second input image, dividing the selected image into at least two motion estimation areas and extracting feature points from the motion estimation areas (S120); arranging blocks with specified sizes to surround the extracted feature points (S130); and estimating a local motion of the blocks (S140) by matching the second input image with the first input image and a global motion in the motion estimation areas (S150).

**FIG. 2**

| | |
|---|---|
| DECOMPOSING INPUT IMAGES | S110 |
| EXTRACTING FEATURE POINTS | S120 |
| ARRANGING BLOCKS | S130 |
| ESTIMATING LOCAL MOTION | S140 |
| ESTIMATING GLOBAL MOTION | S150 |
| COMPOUNDING IMAGES | S160 |

**Description**

The present invention generally relates to a method of forming ultrasound images and a system using the same, and more particularly to a method of estimating hierarchical motion for accurately estimating real-time motion from a sequence of ultrasound images and an ultrasound imaging system using the same.

Ultrasound imaging is a non-invasive technique typically used for examining internal organs. Ultrasound imaging is performed by sending ultrasound waves into the body. The reflected ultrasound waves are recorded and then displayed as a real-time visual image. Since the ultrasound images are displayed in real time, they can show the movements of internal tissues, organs and blood flow.

The ultrasound images are used in various medical applications. Thus, wide-view images (i.e., panoramic images) need to be acquired from a sequence of ultrasound images. These panoramic images display the organs and their adjacent anatomical features in one single image, thereby eliminating the need for physicians to mentally piece together a complete picture of anatomical structures. For example, the panoramic images can display both lobes of the thyroid gland on a single image to thereby provide an accurate image representation of various thyroid disorders.

To acquire the panoramic image, it is essential to accurately estimate motion in sequential images. The known techniques for estimating motion include the gradient technique, pel-recursive technique, block matching technique, etc. In the gradient and pel-recursive techniques, the motion is estimated for every pixel in order to obtain motion information. However, they are complicated and require an enormous amount of calculation. As such, the block matching technique is more widely used than the above techniques. In the block matching technique, the arrangement and sizes of the blocks affect the performance of motion estimation. Specifically, the arrangement of blocks affects the estimation variance of global motion components, whereas the sizes of blocks influence the amount of calculation. In the global motion estimation, it is important to regularly arrange the blocks in their respective motion estimation areas.

However, when the panoramic ultrasound images are obtained by using the block matching technique, the amount of calculation is very large since the blocks must be enlarged to contain features. Further, a speckle noise is examined in the ultrasound images since the phase change occurs due to a medium and the like when the ultrasound waves are reflected. The speckle noise degrades the quality and interpretation of the images. Therefore, it takes a very long time to acquire the panoramic ultrasound images. Thus, it becomes very difficult to obtain accurate panoramic ultrasound images.

The present invention provides a method of estimating hierarchical motion to accurately display a sequence of images in real time by decreasing an amount of calculation for processing sequential images as well as to reduce an error in global motion estimation. Further, the present invention provides an ultrasound imaging system that uses such hierarchical motion estimation method.

In accordance with one aspect of the present invention, there is provided a hierarchical motion estimation method, which includes the following steps: (1) decomposing first and second input images into at least two hierarchical, multi-resolution images; (2) selecting one of the multi-resolution images decomposed from the second input image, dividing the selected image into at least two motion estimation areas and extracting feature points from the motion estimation areas; (3) arranging blocks with specified sizes to surround the extracted feature points; and (4) estimating local motions of the blocks by matching the second input image with the first input image and predicting a global motion in the motion estimation areas.

In accordance with another aspect of the present invention, there is provided an ultrasound imaging system, which includes the following: (1) an image decomposition unit for decomposing first and second ultrasound input images into at least two hierarchical, multi-resolution images; (2) a feature extraction unit for selecting one of the multi-resolution images decomposed from the second input image, the feature extraction unit further being configured to divide the selected image into at least two motion estimation areas and extract feature points from the motion estimation areas; (3) a block arrangement unit for arranging blocks with specified sizes to surround the extracted feature points; and (4) a motion estimation unit for estimating local motions of the blocks by matching the second input image with the first input image to predict a global motion in the motion estimation areas.

Preferably, the blocks are arranged so as to be separated from each other. Further, the input image may be decomposed into hierarchical, multi-resolution images according to the following equation:

$$f_k(i,\ j) = \frac{1}{4} \sum_{l=0}^{1}\ \sum_{m=0}^{1} f_{k+1}(2i+l,\ 2j+m)$$

wherein $f_k(i,\ j)$ represents a pixel at a position (i, j) in an image of k-level resolution, and i and j (i, j = 0, ..., $2^k$ -1) are coordinates of pixels. Furthermore, the feature points may be extracted from an image having the lowest resolution. The feature points may be extracted according to the following equation:

$$G(i,j) = |N(x,y)| - kTrace^2 N(x,y)$$

wherein $N(x,y) = \begin{bmatrix} I^2_x & I_xI_y \\ I_xI_y & I^2_y \end{bmatrix}$, k is a weight, and $I_x$ and $I_y$ are gradient magnitudes along x- and y-axes, respectively.

The input image may be an ultrasound image, and the first and second input image may be sequential in time.

In accordance with still another aspect of the present invention, there is provided a method for forming a panoramic image from sequential images by using hierarchical motion estimation in an ultrasound imaging system, which includes the following steps: a) decomposing each of first and second input images occurring sequentially into at least two hierarchical multi-resolution images; b) dividing a lowest resolution image of the multi-resolution images decomposed from the second input image into at least two motion estimation areas and extracting feature points from the motion estimation areas, respectively; c) arranging blocks having a specified size to surround the extracted feature points, respectively; d) setting a search window on a lowest resolution image of the multi-resolution images decomposed from the first input image; e) matching the blocks with the lowest resolution image of the first input image within the search window to estimate local motion in the blocks; f) estimating global motion in the motion estimation areas by using the estimated local motion; and h) compounding the first and second input images on the basis of the estimated global motion to form a panoramic image.

In accordance with the present invention, since the feature points are extracted by using the multi-resolution images, the error caused by a speckle noise can be reduced and the calculation time can be decreased. Further, in accordance with the present invention, since the blocks are separately arranged after dividing into the motion estimation areas, the calculation time can be reduced without affecting accuracy.

The above and other objects and features of the present invention will become apparent from the following descriptions of preferred embodiments given in conjunction with the accompanying drawings, in which:

Fig. 1 is a block diagram showing the schematic configuration of an ultrasound imaging system constructed in accordance with the preferred embodiment of the present invention;
Fig. 2 is a flow chart schematically showing a hierarchical motion estimation method in accordance with the preferred embodiment of the present invention;
Figs. 3A and 3B show a multi-resolution image pyramid and a mapping process, respectively;
Figs. 4A and 4B are 9x9 kernel matrices, which are x-axial and y-axial edge operators, respectively;
Fig. 5A shows the block arrangement in the hierarchical motion estimation method in accordance with the preferred embodiment of the present invention;
Fig. 5B shows a modification of the block arrangement; and
Figs. 6A and 6B are ultrasound images for explaining an image compounding step in the hierarchical motion estimation method in accordance with the preferred embodiment of the present invention.

Hereinafter, the preferred embodiments of the present invention will be described with reference to the accompanying drawings.

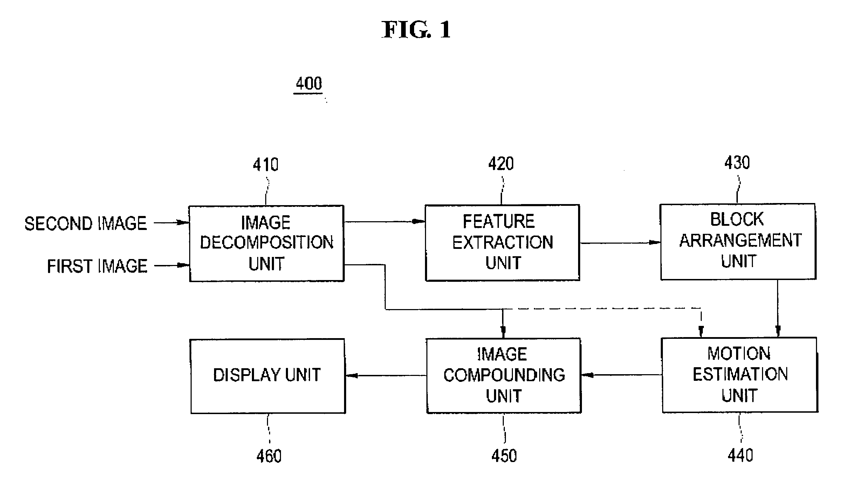

Fig. 1 is a block diagram showing the schematic configuration of an ultrasound imaging system, which is constructed in accordance with the preferred embodiment of the present invention.

Referring to Fig. 1, an ultrasound imaging system 400 includes: an image decomposition unit 410; a feature extraction unit 420; a block arrangement unit 430; a motion estimation unit 440; an image compounding unit 450; and a display unit 460.

To estimate the motion with first and second ultrasound input images, the image decomposition unit 410 decomposes the first and second ultrasound input images into at least two hierarchical, multi-resolution images. The feature extraction unit 420 selects an arbitrary image among the multi-level resolution images decomposed from the second input image. It then divides the selected image into at least two motion estimation areas and extracts feature points from the motion estimation areas. Preferably, the lowest resolution image is selected and the feature points are extracted from the motion estimation areas in the lowest resolution image. The block arrangement unit 430 arranges blocks with specified sizes to surround the extracted feature points. Preferably, the blocks are arranged in a way so as not to overlap each other. The motion estimation unit 440 estimates local motions of the blocks by matching the second input image with the first input image and predicts a global motion in the motion estimation areas. The image compounding unit 450 creates a panoramic image by compounding the sequential images after the global motion estimation. Image compounding is performed in order to produce a seamless image by controlling the brightness information in pixels in an overlapping area of the sequential images. The display unit 460 displays a compound image transferred from a frame buffer (not shown) on a monitor (not shown).

The hierarchical motion estimation method, which is in accordance with the preferred embodiment of the present invention, will now be described below.

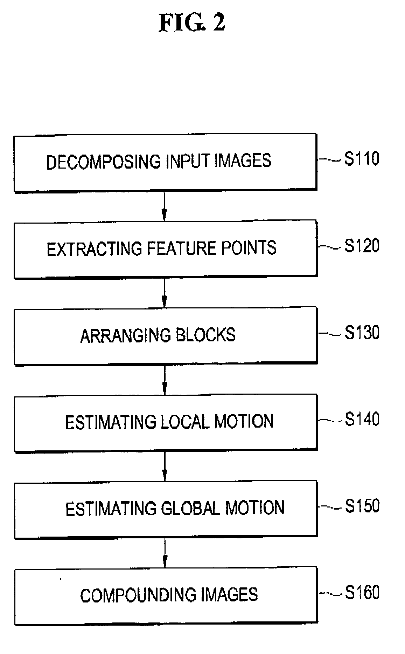

Fig. 2 is a flow chart schematically showing the hierarchical motion estimation method of the present invention, which includes: step S110 for decomposing input images; step S120 for extracting feature points; step S130 for arranging blocks; step S140 for estimating local motions; step S150 for estimating a global motion; and step S160 for compounding images.

At step S110, the image decomposition unit 410 decomposes first and second input images occurring sequentially in time into at least two hierarchical, multi-resolution images. The first and second input images correspond to previous and current images, respectively.

The input image is decomposed into hierarchical, multi-resolution images according to the following equation:

$$f_k(i, j) = \frac{1}{4} \sum_{l=0}^{1} \sum_{m=0}^{1} f_{k+1}(2i+l, 2j+m) \quad \dots\dots\dots\dots\dots\dots \quad (1)$$

wherein $f_k(i, j)$ represents a pixel at a position (i, j) in an image of k-level resolution, and i and j (i, j = 0, ..., $2^k$-1) are coordinates of pixels.

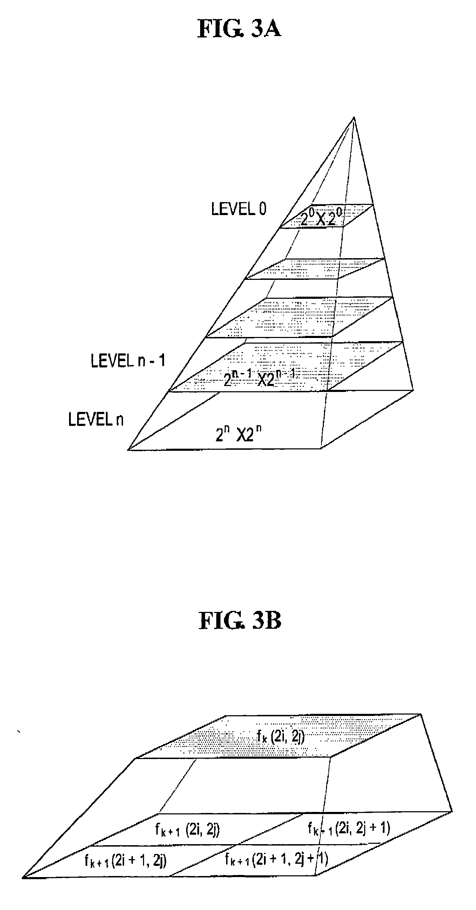

Fig. 3A shows a multi-resolution image pyramid represented by Eq. (1). The multi-resolution image pyramid includes a series of images having $2^k \times 2^k$ resolution (k = 0, ..., $n$-1). The image of $2^n \times 2^n$ resolution at the bottom of the pyramid is an original image decomposed into hierarchical, multi-resolution images in the bottom-up manner. Fig. 3B shows a mapping process, wherein pixel $f_k(i, j)$ at k level is produced from four pixels $f_{k+1}(2i,2j)$, $f_{k+1}(2i,2j+1)$, $f_{k+1}(2i+1,2j)$ and $f_{k+1}(2i+1, 2j+1)$ adjacent to each other at k+1 level. At this time, the mapping function from an upper level to a lower level is a low-pass operation, whereby high frequency components are suppressed to obtain a lower resolution image.

At step S120, the feature extraction unit 420 selects an arbitrary image among the multi-level resolution images decomposed from the second input image. It then divides the selected image into at least two motion estimation areas and extracts feature points from the motion estimation areas.

Preferably, the lowest resolution image is selected and the feature points are extracted from the motion estimation areas in the lowest resolution image. Accordingly, since a speckle noise is reduced in the input image, the feature points can be more accurately extracted while the calculation time is reduced.

The feature points are extracted from the motion estimation areas by using the Harris-Stephen corner detection method, which is represented by the following equation:

$$G(i,j) = |N(x,y)| - kTrace^2 N(x,y) \quad \dots\dots\dots\dots\dots \quad (2)$$

wherein

$$N(x,y) = \begin{bmatrix} I^2_x & I_x I_y \\ I_x I_y & I^2_y \end{bmatrix};$$

k is a weight (0.04 is generally used); $I_x$ is a gradient magnitude along an x-axis; and $I_y$ is a gradient magnitude along a y-axis.

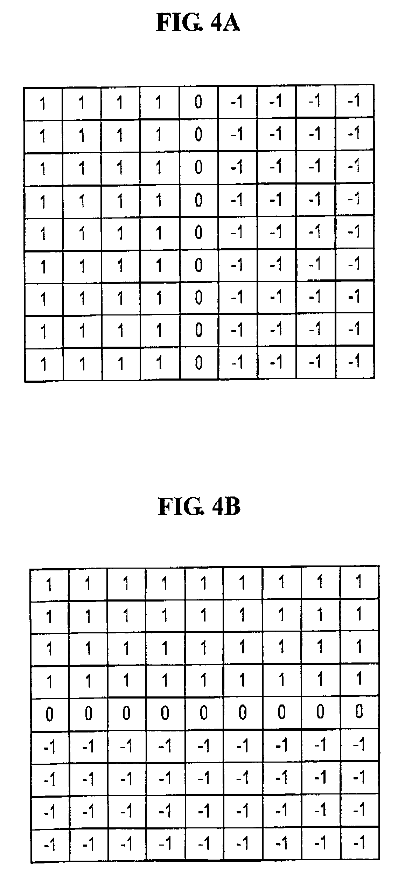

In each motion estimation area, the x-axial and y-axial gradient magnitude $I_x$ and $I_y$ at each pixel are calculated by using 9x9 kernel matrices, i.e., x-axial and y-axial edge operators, respectively (shown in Figs. 4A and 4B). Then, the corner where both the x-axial and y-axial gradient magnitudes (edge strengths) have large values is extracted as a feature point in each motion estimation area by using Eq. (2). At step S130, the block arrangement unit 430 arranges blocks with specified sizes to surround the extracted feature points.

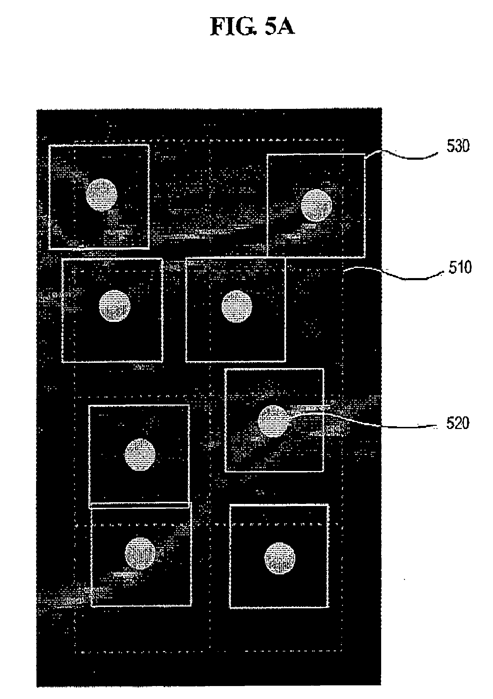

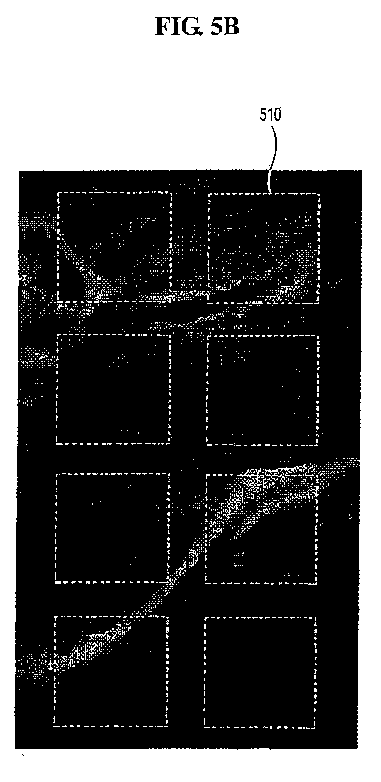

Fig. 5A shows the block arrangement in the hierarchical motion estimation method. Referring to Fig. 5A, blocks 530 are arranged to contain the extracted feature points 520 in the motion estimation areas 510. At this time, when the feature points are close to each other, there may be an error in the global motion estimation due to the blocks overlapping each other. Thus, as shown in Fig. 5B, the motion estimation areas 510 are preferably separated from each other at step S120 in order to prevent the blocks from overlapping each other.

At step S140, the motion estimation unit 440 estimates local motions of the blocks by matching the second input image with the first input image. That is, the motion of blocks is estimated by estimating the positions of the previous image, which correspond to the blocks in the current image.

For the local motion estimation, block matching is performed to find a reference block with the best match compared to the block in the current image. The Sum Absolute Difference (SAD), which is the sum of absolute differences between the values of corresponding pixels between each block in the current image and a reference block, is investigated within a search window in the previous image. The best match is considered to occur when the SAD value is at a minimum.

Generally, the search window is set to have a horizontal length longer than a longitudinal length due to a large motion in a horizontal direction in the sequence of ultrasound images. The search window is sufficiently large to contain motion between the ultrasound images. However, it should be noted that the larger the search window gets, the larger the amount of calculation becomes.

In this embodiment, by using the above-described lowest resolution image, the search window can be made to be smaller without compromising accuracy while reducing the amount of calculation.

At step S 150, the global motion is estimated by analyzing the local motions of the blocks. At step S160, the image compounding unit 450 creates a panoramic image by compounding the sequential images after the global motion estimation.

The techniques for combining the images include image growing, recursive spatial compounding, ramp compounding and the like. The image growing technique only puts new pixel data in a non-overlapping part to a panoramic image buffer. The recursive spatial compounding technique recursively averages a new image frame with the existing panoramic image. The ramp compounding technique provides weight ramps for both the new image frame and the existing panoramic image in an overlapping area.

In case of image growing technique, the continuity of the compound image can be enhanced by averaging the frames in the overlapping area through the following equation:

$$G(x,y,t) = \frac{G(x,y,t-1) + I(x,y,t)}{2} \quad \dots\dots\dots\dots\dots\dots\dots\dots\dots \ (3)$$

wherein G and I represent a panoramic image and an ultrasound image, respectively; x and y are coordinatcs: and t is a frame number.

In this embodiment, the center of rotation in the overlapping area is modified by estimating a rotation angle. Further, a frame averaging method is employed to reduce the discontinuity between the new image frame and the existing panoramic image.

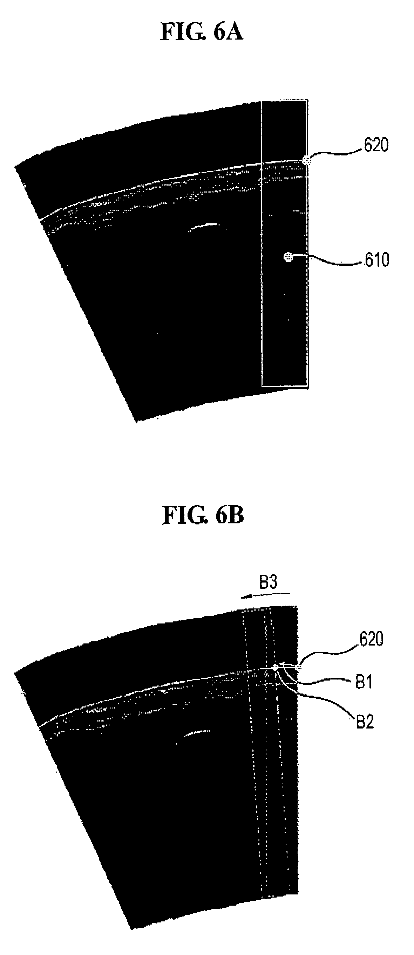

Figs. 6A and 6B are ultrasound images showing skin parts to explain the image compounding step in the hierarchical motion estimation method of the present invention.

In the image compounding step, the portions of respective images are combined without combining the entire images. As shown in Fig. 6A, the center of rotation is changed from the center 610 of overlapping area to a skin position 620. As shown in Fig. 6B, the changed center of rotation (skin position 620) is then moved by an estimated motion in the x-axial direction (B1) and moved to a skin position in the y-axial direction (B2). Finally, the new image frame is combined with the existing panoramic image by rotating it around the skin position (B3).

While the present invention has been described and illustrated with respect to a preferred embodiment of the invention, it will be apparent to those skilled in the art that variations and modifications are possible without deviating from the broad principles and teachings of the present invention, which should be limited solely by the scope of the claims appended hereto.

**Claims**

1. A method of estimating a hierarchical motion, comprising:

decomposing a first input image and a second input image into at least two hierarchical, multi-resolution images;

selecting one of the multi-resolution images decomposed from the second input image, dividing the selected image into at least two motion estimation areas and extracting feature points from the motion estimation areas; arranging blocks with specified sizes to surround the extracted feature points; and estimating a local motion of the blocks by matching the second input image with the first input image and a global motion in the motion estimation areas.

2. The method of Claim 1, wherein the blocks are arranged in a manner so as to be separated from each other.

3. The method of Claim 1, wherein the input image is decomposed into hierarchical, multi-resolution images according to the following equation:

$$f_k(i,\ j)=\frac{1}{4}\sum_{l=0}^{1}\ \sum_{m=0}^{1}f_{k+1}(2i+l,\ 2j+m)$$

wherein $f_k(i,j)$ represents a pixel at a position (i,j) in an image of k-level resolution, and i and j (i, j = 0, ..., $2^k$ -1) are coordinates of pixels.

4. The method of Claim 1, wherein the feature points are extracted from an image with a lowest resolution.

5. The method of Claim 1, wherein the feature points are extracted according to the following equation:

$$G(i,j)=\left|N(x,y)\right|-kTrace^2N(x,y)$$

wherein

$$N(x,y) = \begin{bmatrix} I^2{}_x & I_xI_y \\ I_xI_y & I^2{}_y \end{bmatrix},$$

k is a weight, and $I_x$ and $I_y$ are gradient magnitudes along an x-axis and a y-axis, respectively.

6. The method of Claim 1, wherein the input image is an ultrasound image.

7. The method of Claim 1. wherein the first and second input images are sequential in time.

8. An ultrasound imaging system, comprising:

an image decomposition unit for decomposing first and second ultrasound input images into at least two hierarchical, multi-resolution images;
a feature extraction unit for selecting one of the multi-resolution images decomposed from the second input image, the feature extraction unit being configured to divide the selected image into at least two motion estimation areas and extract feature points from the motion estimation areas;
a block arrangement unit for arranging blocks with specified sizes to surround the extracted feature points; and
a motion estimation unit for estimating a local motion of the blocks by matching the second input image with the first input image and a global motion in the motion estimation areas.

9. The system of Claim 8, wherein the blocks are arranged in a manner so as to be separated from each other.

10. A method for forming a panoramic image from sequential images by using hierarchical motion estimation in an ultrasound imaging system, comprising:

decomposing each of first and second input images occurring sequentially into at least two hierarchical multi-resolution images;

dividing a lowest resolution image of the multi-resolution images decomposed from the second input image into at least two motion estimation areas and extracting feature points from the motion estimation areas, respectively;

arranging blocks having a specified size to surround the extracted feature points, respectively;

setting a search window on a lowest resolution image of the multi-resolution images decomposed from the first input image;

matching the blocks with the lowest resolution image of the first input image within the search window to estimate local motion in the blocks;

estimating global motion in the motion estimation areas by using the estimated local motion; and

compounding the first and second input images on the basis of the estimated global motion to form a panoramic image.

**FIG. 1**

400

SECOND IMAGE →

FIRST IMAGE →

| IMAGE DECOMPOSITION UNIT | FEATURE EXTRACTION UNIT | BLOCK ARRANGEMENT UNIT |

410   420   430

DISPLAY UNIT ← IMAGE COMPOUNDING UNIT ← MOTION ESTIMATION UNIT

460   450   440

# FIG. 2

DECOMPOSING INPUT IMAGES ——S110

EXTRACTING FEATURE POINTS ——S120

ARRANGING BLOCKS ——S130

ESTIMATING LOCAL MOTION ——S140

ESTIMATING GLOBAL MOTION ——S150

COMPOUNDING IMAGES ——S160

## FIG. 3A

LEVEL 0    $2^0 X 2^0$

LEVEL n - 1    $2^{n-1} X 2^{n-1}$

LEVEL n    $2^n X 2^n$

## FIG. 3B

$f_k (2i, 2j)$

$f_{k+1} (2i, 2j)$    $f_{k+1} (2i, 2j + 1)$

$f_{k+1} (2i + 1, 2j)$    $f_{k+1} (2i + 1, 2j + 1)$

## FIG. 4A

| 1 | 1 | 1 | 1 | 0 | -1 | -1 | -1 | -1 |
|---|---|---|---|---|----|----|----|----|
| 1 | 1 | 1 | 1 | 0 | -1 | -1 | -1 | -1 |
| 1 | 1 | 1 | 1 | 0 | -1 | -1 | -1 | -1 |
| 1 | 1 | 1 | 1 | 0 | -1 | -1 | -1 | -1 |
| 1 | 1 | 1 | 1 | 0 | -1 | -1 | -1 | -1 |
| 1 | 1 | 1 | 1 | 0 | -1 | -1 | -1 | -1 |
| 1 | 1 | 1 | 1 | 0 | -1 | -1 | -1 | -1 |
| 1 | 1 | 1 | 1 | 0 | -1 | -1 | -1 | -1 |
| 1 | 1 | 1 | 1 | 0 | -1 | -1 | -1 | -1 |

## FIG. 4B

| 1 | 1 | 1 | 1 | 1 | 1 | 1 | 1 | 1 |
|----|----|----|----|----|----|----|----|----|
| 1 | 1 | 1 | 1 | 1 | 1 | 1 | 1 | 1 |
| 1 | 1 | 1 | 1 | 1 | 1 | 1 | 1 | 1 |
| 1 | 1 | 1 | 1 | 1 | 1 | 1 | 1 | 1 |
| 0 | 0 | 0 | 0 | 0 | 0 | 0 | 0 | 0 |
| -1 | -1 | -1 | -1 | -1 | -1 | -1 | -1 | -1 |
| -1 | -1 | -1 | -1 | -1 | -1 | -1 | -1 | -1 |
| -1 | -1 | -1 | -1 | -1 | -1 | -1 | -1 | -1 |
| -1 | -1 | -1 | -1 | -1 | -1 | -1 | -1 | -1 |

# FIG. 5A

# FIG. 5B

510

## FIG. 6A

## FIG. 6B

**European Patent**
**Office**

# EUROPEAN SEARCH REPORT

Application Number

EP 06 01 3450

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 6 456 731 B1 (CHIBA NAOKI [JP] ET AL) 24 September 2002 (2002-09-24) * abstract * * figures 5,6 * * column 2, line 30 - line 45 * * column 10, line 65 - column 11, line 8 * * column 13, line 8 - column 14, line 43 * | 1-10 | INV. G06T7/20 ADD. A61B8/00 |
| Y | ZHANG Z ET AL: "A ROBUST TECHNIQUE FOR MATCHING TWO UNCALIBRATED IMAGES THROUGH THE RECOVERY OF THE UNKNOWN EPIPOLAR GEOMETRY" ARTIFICIAL INTELLIGENCE, ELSEVIER SCIENCE PUBLISHER B.V., AMSTERDAM, NL, vol. 78, no. 1/2, 1995, pages 87-119, XP001148319 ISSN: 0004-3702 * abstract * * page 103 * | 1-10 | |
| A | US 5 782 766 A (WENG LEE [US] ET AL) 21 July 1998 (1998-07-21) * abstract * * column 2, line 63 - column 3, line 12 * * figure 2 * | 1-10 | TECHNICAL FIELDS SEARCHED (IPC) G06T H04N |
| A | KELLER Y ET AL: "FAST GRADIENT METHODS BASED ON GLOBAL MOTION ESTIMATION FOR VIDEO COMPRESSION" IEEE TRANSACTIONS ON CIRCUITS AND SYSTEMS FOR VIDEO TECHNOLOGY, IEEE SERVICE CENTER, PISCATAWAY, NJ, US, vol. 13, no. 4, April 2003 (2003-04), pages 300-309, XP001158239 ISSN: 1051-8215 * abstract * * sequences III-A * | 1-10 | |

The present search report has been drawn up for all claims

| Place of search | Date of completion of the search | Examiner |
|---|---|---|
| Munich | 10 October 2006 | Eveno, Nicolas |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 06 01 3450

This annex lists the patent family members relating to the patent documents cited in the above-mentioned European search report.
The members are as contained in the European Patent Office EDP file on
The European Patent Office is in no way liable for these particulars which are merely given for the purpose of information.

10-10-2006

| Patent document cited in search report | | Publication date | Patent family member(s) | | Publication date |
|---|---|---|---|---|---|
| US 6456731 | B1 | 24-09-2002 | JP | 3435084 B2 | 11-08-2003 |
| | | | JP | 11339021 A | 10-12-1999 |
| | | | US | 2003058945 A1 | 27-03-2003 |
| US 5782766 | A | 21-07-1998 | US | 5899861 A | 04-05-1999 |

EPO FORM P0459

For more details about this annex : see Official Journal of the European Patent Office, No. 12/82